# EUROPÄISCHE PATENTANMELDUNG

(11) **EP 0 530 690 A2**
(43) Veröffentlichungstag der Anmeldung: **10.03.1993**
(21) Anmeldenummer: 92114698.1
(22) Anmeldetag: 28.08.1992
(51) Int. Cl.: C07D 471/08

(54) **1-Azabicyclo(4,3,1)decan**

(30) Priorität: 06.09.1991 DE 4129764
(71) Anmelder: BASF Aktiengesellschaft, D-67063 Ludwigshafen (DE)
(72) Erfinder: Neuhauser, Horst, Dr., W-6724 Dudenhofen (DE); Siegel, Hardo, Dr., W-6720 Speyer (DE); Koehler, Ulrich, Dr., W-6800 Mannheim 51 (DE)

(57) **Zusammenfassung**

1-Azabicyclo[4,3,1]decan der Formel I 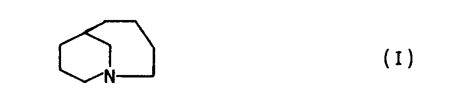 sowie ein Verfahren zu dessen Herstellung, indem man ein Amin der Formel II 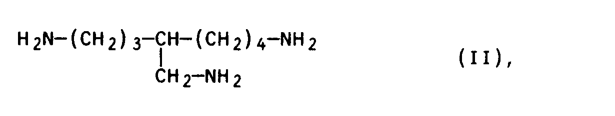 und/oder III 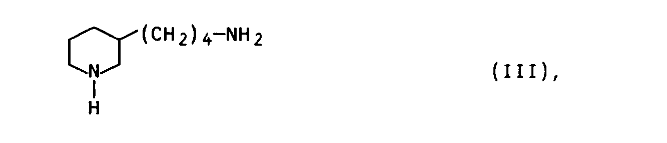 an sauren Katalysatoren umsetzt.

## Beschreibung

Diese Erfindung betrifft ein neues Amin 1-Azabicyclo[4,3,1]decan sowie Verfahren zu dessen Herstellung.

Sterisch gehinderte tertiäre Amine wie z.B. 1,4-Diazabicyclo[2,2,2]octan, 1,5-Diazabicyclo[4,3,0]non-5-en oder 1,4-Diazabicyclo-[5,4,0]undec-7-en mit unterschiedlicher sterischer Hinderung sind bekannt. Diese sind Basen mit abgestufter Basizität. Sie eignen sich u.a. als Katalysatoren zur Herstellung von Polyurethanen.

Es lag daher die Aufgabe zugrunde, ein neues sterisch gehindertes tertiäres Amin zugänglich zu machen, das bezüglich sterischer Hinderung, der Basizität bzw. des Geruches verbesserte Eigenschaften aufweist.

Demgemäß wurde ein neues Amin, das 1-Azabicyclo[4,3,1]decan sowie Verfahren zur dessen Herstellung gefunden.

Das 1-Azabicyclo[4,3,1]decan läßt sich wie folgt herstellen:
Ein Triamin der Formel II oder ein Diamin der Formel III oder eine beliebige Mischung aus beiden Komponenten II und III kann bei Temperaturen von 50 bis 600 °C, d.h. bei Temperaturen von 50 bis 200 °C, bevorzugt 70 bis 150°C in der Füssigphase oder bevorzugt in der Gasphase bei Temperaturen von 200 bis 600°C, vorzugsweise von 300 bis 550°C und einem Druck von 0,01 bis 20 bar, vorzugsweise bei Normaldruck (Atmosphärendruck) in Gegenwart eines sauren Katalysators umgesetzt werden.

Als saure Katalysatoren eignen sich bevorzugt saure Heterogenkatalysatoren wie Zeolithe wie z.B. Aluminiumsilikatzeolithe, Borsilikatzeolithe, Eisensilikatzeolithe, Galliumsilikatzeolithe und Silikalithe des Pentasil-, Faujasit-, X-, Y-Typs. Es kommen u.a. ZSM-5, ZSM-11, ZBM-11, NU-1 und NU-3 in Frage. Besonders bevorzugt unter den sauren Heterogenkatalysatoren sind saure Metallsalze und saure Metalloxide wie Phosphate, Hydrogenphosphate, Dihydrogenphosphate, Pyrophosphate und beliebige andere Phosphate der Elemente Ca, Ba, Sr, Al, Fe und Zr mit und ohne Zusätze von Si0₂, Ti0₂, A1₂0₃ (auch als Trägermaterial) und Oxide der Elemente Mo und W.

Die Reaktionszeiten betragen normalerweise 0,1 bis 90 Sekunden. Die Katalysatorbelastung kann 0,01 bis 50 kg, vorzugsweise 0,05 bis 20 kg Edukt II und/oder III pro kg Katalysator.

### Beispiel 1

Ein elektrisch beheizbarer Festbettreaktor wird mit 40 ml AIP0₄-Katalysator gefüllt. Bei einer Kontakttemperatur von 400°C werden 10 ml/h Triamin 1 dampfförmig bei Normaldruck in Gegenwart von 20 I/h Stickstoff über den Katalysator geleitet. Das Reaktionsgemisch wird kondensiert und gaschromatographisch analysiert.
Triaminumsatz: 90 %
Produktselektivität: 52 %

### Beispiel 2

Ein elektrisch beheizbarer Festbettreaktor wird mit 40 ml SrHP0₄-Katalysator gefüllt. Bei einer Kontakttemperatur von 475 °C werden 10 ml/h Diamin II dampfförmig bei Normaldruck in Gegenwart von 10 I/h Stickstoff über den Katalysator geleitet. Das Reaktionsgemisch wird kondensiert und gaschromatographisch analysiert.
Diaminumsatz: 80 %
Produktselektivität: 48 %

### Beispiel 3

Ein elektrisch beheizbarer Festbettreaktor wird mit 40 ml AIP0₄-Katalysator gefüllt. Bei einer Kontakttemperatur von 425°C werden 10 ml/h Diamin II dampfförmig bei Normaldruck in Gegenwart von 30 I/h Stickstoff über den Katalysator geleitet. Das Reaktionsgemisch wird kondensiert und gaschromatographisch analysiert.
Diaminumsatz: 91 %
Produktselektivität: 65 %
Charakterisierung von 1-Azabicyclo[4,3,1]decan:
Schmelzpunkt: 49 °C
Siedepunkt: 83°C/15 mbar
   ¹H-NMR (CDCl₃): d = 1,2 - 2,05 (m, 11 H) d = 2,75 - 3,05 (m, 6H)
   ¹³C-NMR (CDCl₃): C1: d = 51,93 (t) C6: d = 33,00 (t)
      C2: d = 32,21 (t) C7: d = 21,10 (t)
      C3: d = 27,22 (t) C8: d = 52,80 (t)
      C4: d = 33,11 (t) C9: d = 56,89 (t)
      C5: d = 31,00 (d)

## Patentansprüche

1. 1-Azabicyclo[4,3,1]decan der Formel I

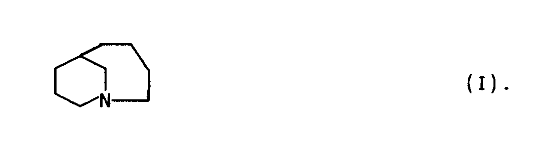

2. Verfahren zur Herstellung von 1-Azabicyclo[4,3,1]decan nach Anspruch 1, dadurch gekennzeichnet, daß man ein Amin der Formel II 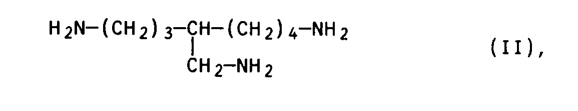 und/oder III 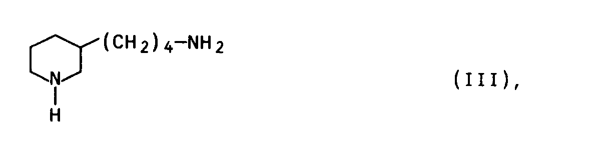 an sauren Katalysatoren umsetzt.

3. Verfahren zur Herstellung von 1-Azabicyclo[4,3,1]decan nach Anspruch 1, dadurch gekennzeichnet, daß man die Umsetzung an sauren Heterogenkatalysatoren durchführt.

4. Verfahren zur Herstellung von 1-Azabicyclo[4,3,1]decan nach Anspruch 1, dadurch gekennzeichnet, daß man in der Gasphase arbeitet.
